## Europäisches Patentamt
## European Patent Office
## Office européen des brevets

(19)

(11) Numéro de publication: **0 150 672**
A1

## (12) DEMANDE DE BREVET EUROPEEN

(21) Numéro de dépôt: 84810623.3

(22) Date de dépôt: 17.12.84

(51) Int. Cl.⁴: **G 01 F 1/66**, G 01 P 5/00, A 61 B 8/06

(30) Priorité: 27.01.84 CH 391/84

(71) Demandeur: **Novatec S.A., 7, rue de Longemalle, CH-1020 Renens (CH)**

(43) Date de publication de la demande: 07.08.85 Bulletin 85/32

(72) Inventeur: **Meister, Jean-Jacques, Chemin Ochettaz 7, CH-1025 St-Sulpice (CH)**

(84) Etats contractants désignés: **AT BE CH DE FR GB IT LI LU NL SE**

(74) Mandataire: **Vuille, Roman et al, c/o KIRKER & Cie S.A. 14, rue du Mont-Blanc Case Postale 872, CH-1211 Genève 1 (CH)**

(54) Procédé et appareil de détermination, par échographie Doppler, de la vitesse d'écoulement et du débit d'un fluide dans un conduit.

(57) Deux émetteurs-récepteurs (3, 4) de trains d'ondes solidaires l'un de l'autre sont montés sur un support pour être orientables à volonté par rapport à un conduit (2) dans lequel circule un fluide dont on veut connaître la vitesse et le débit. Un émetteur-récepteur (3) est amené en position de perpendicularité de son axe par rapport à l'axe du conduit (2). Des moyens (14) sont prévus pour mesurer le temps de transit entre l'émission d'un train d'ondes par cet émetteur-récepteur (3) et la réception du train réfléchi. Des moyens de calcul (13) calculent le diamètre et la section droite du conduit. Des moyens (11) calculent la vitesse instantanée du fluide en différents endroits de cette section et le débit instantané dans le conduit (2).

EP 0 150 672 A1

### Procédé et appareil de détermination, par échographie Doppler, de la vitesse d'écoulement et du débit d'un fluide dans un conduit.

La présente invention se rapporte à un procédé et un appareil de détermination, par échographie Doppler, de la vitesse d'écoulement et du débit d'un fluide dans un conduit, par exemple du sang dans un vaisseau du corps.

On connaît déjà des procédés et des appareils d'échographie Doppler acoustique que l'on utilise pour observer l'écoulement du sang dans des vaisseaux, en dirigeant un faisceau d'ondes ultrasonores vers une région du corps que l'on désire examiner (brevets USA 3.550.070 et 3.554.030 par ex.). Cette méthode simple permet de déterminer si, dans la région explorée, le sang circule. Mais si on ne peut pas localiser exactement un vaisseau d'une certaine importance et que de ce fait le faisceau n'est pas dirigé exactement sur lui, on ne peut faire aucune détermination de valeur de vitesse d'écoulement du sang et encore moins de débit sanguin. Ces appareils connus ne sont utiles que si on les utilise sur certaines régions du corps (bras et jambes par exemple) où l'on connaît l'emplacement exact de vaisseaux importants situés dans le voisinage immédiat de la peau. Dans ces cas, où l'on sait que l'échographie porte effectivement sur un vaisseau d'une certaine importance, on peut, par analyse de l'effet Doppler, calculer une certaine valeur de vitesse d'écoulement qui donne une information qualitative quant à la circulation dans le vaisseau considéré. Toutefois, ces appareils connus ne permettent pas de déterminer la vitesse réelle et encore moins le débit exact, car on ne connaît ni l'angle précis entre l'axe du faisceau et l'axe du vaisseau à l'endroit d'observation, ni non plus la dimension réelle de ce vaisseau, que l'on doit simplement admettre en se basant sur des moyennes connues (voir par exemple brevet USA 3.554.030 Peronneau).

Or comme le plus souvent on cherche à établir un diagnostic quant à l'état des vaisseaux sanguins d'un patient (degré de colmatage des parois notamment) il est de toute importance de disposer d'informations aussi exactes que possible en ce qui concerne le débit sanguin instantané réel et la répartition des vitesses le long d'un diamètre de la section transversale du vaisseau observé. Les méthodes et appareils connus ne permettent pas de déterminer ce débit exact et cette répartition des vitesses.

La présente invention vise à résoudre ce problème et elle a pour objet un procédé selon la revendication 1 et un appareil selon la revendication 3.

Le dessin annexé représente, à titre d'exemple, une forme d'exécution de l'appareil selon l'invention, illustrant une mise en oeuvre du procédé conforme à l'invention.

Fig. 1 est un schéma-bloc de cette forme d'exécution.

Fig. 2 et 3 sont des diagrammes explicatifs relatifs à la fig. 1.

Fig. 4 est une vue en perspective du dispositif mécanique porteur des émetteurs-récepteurs d'ondes ultrasonores indiqués schématiquement en 3, 4 sur la fig. 1.

Fig. 5 est une vue analogue à la fig. 4 mais relative à une variante.

Fig. 6 est un schéma du dispositif représenté par le bloc 7 sur fig. 1.

Fig. 7 et 8 sont des diagrammes explicatifs.

Fig. 9 est une vue en perspective illustrant le profil des vitesses d'un fluide dans un conduit.

Fig. 10 est un diagramme illustrant les deux effets Doppler se manifestant dans un conduit à paroi élastique dans lequel circule un fluide soumis à des pulsations.

Fig. 11 est un diagramme explicatif.

Fig. 12 est un schéma du dispositif représenté par le bloc 9 de fig. 1.

Fig. 13 est un diagramme explicatif.

Fig. 14 et 15 sont des diagrammes comparatifs du débit obtenu avec l'appareil selon fig. 1 et les appareils connus.

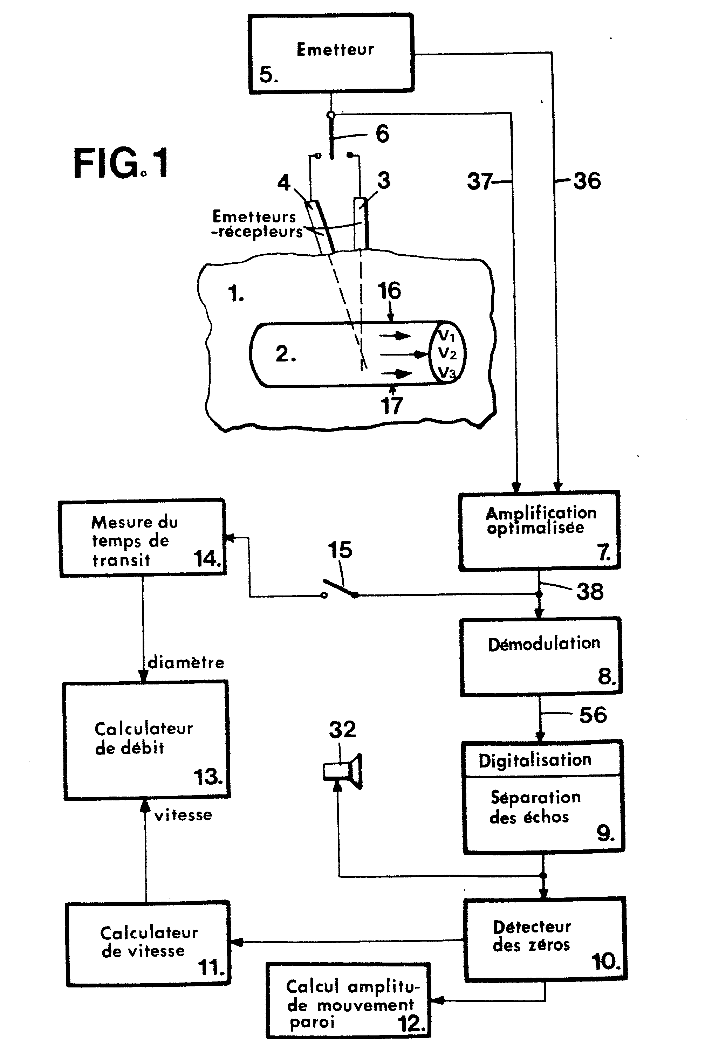

La figure 1 représente schématiquement l'ensemble de la première forme d'exécution qui est un appareil pour la détermination de la vitesse d'écoulement et du débit du sang dans un vaisseau sanguin. On verra que cet appareil permet de déterminer d'autres paramètres.

On a indiqué en 1 un tissu vivant à l'intérieur duquel se trouve un vaisseau sanguin 2 dans lequel le sang circule avec des vitesses qui sont maximum dans la région axiale et minimum au voisinage des parois. Ces vitesses différentes sont schématisées par des vecteurs $v_1$, $v_2$, $v_3$. On a représenté en 3 et 4 deux émetteurs-récepteurs d'ultrasons. L'émetteur-récepteur 3 est orienté perpendiculairement à l'axe du vaisseau 2 dans la région considérée tandis que l'émetteur-récepteur 4 est dirigé obliquement par rapport à cet axe. Les faisceaux d'ultrasons de 3 et 4 font entre eux un angle fixe connu et le plan contenant les axes de ces deux faisceaux passe par l'axe du vaisseau 2. En 5 se trouve un émetteur                 prévu pour alimenter soit l'émetteur-récepteur 3, soit l'émetteur-récepteur 4, comme on le verra plus loin, grâce à un commutateur 6. Les émetteurs-récepteurs 3 et 4 sont destinés à permettre la détermination des vitesses $v_1$, $v_2$, $v_3$ par procédé connu d'échographie Doppler.

Les signaux d'écho Doppler provenant de 3 et 4 sont envoyés à un dispositif 7 d'amplification optimalisée qui sera décrit en détail plus loin. En 8 est un démodulateur, en 9 un dispositif de digitalisation des signaux démodulés provenant de 8, et un dispositif de séparation des échos qui sera décrit plus loin. En 10 se trouve un détecteur de zéro qui sera également décrit plus loin. 10 comporte une sortie allant à un calculateur 11 servant à calculer les vitesses du sang dans le vaisseau 2. Une autre sortie de 10 va à un dispositif de calcul de l'amplitude du mouvement des parois du vaisseau 2 que l'on décrira dans la suite. La sortie de 11 va à un calculateur 13 calculant le débit sanguin. En 14 se trouve un dispositif de mesure du temps de transit des ultrasons émis par 3 et faisant retour à celui-ci. Ce temps de transit ainsi mesuré est envoyé au calculateur 13 qui, sur la base des temps de transit mesurés, calcule la section transversale du vaisseau 2. Un interrupteur 15 permet de mettre en fonction le dispositif 14.

L'émetteur 5 émet des signaux électriques sinusoï-

daux par trains de durée et d'amplitude réglables. Le démodulateur 8 peut être du type décrit dans la publication suivante : McLeod, F.D., Anliker, M. : A multiple gate pulse Doppler flowmeter : Proc. IEEE Ultrasonics, Miami, 1971.

Le calculateur 11 calcule les vitesses locales du sang à différentes profondeurs dans le vaisseau 2, vitesses qui sont proportionnelles à la fréquence Doppler. Le mode de calcul peut être celui décrit par exemple dans le brevet USA 3 914 999 de Grandchamp. Le dispositif 14 sert à détecter l'arrivée de l'écho dû à la réflexion du faisceau émis par 3 contre le côté 16 du vaisseau 2 et contre le côté opposé 17 de ce vaisseau, ce qui permettra de calculer le diamètre du vaisseau. Le brevet USA 3 514 747 explique comment on peut déterminer le temps de transit en mesurant le temps qui sépare deux échos successifs.

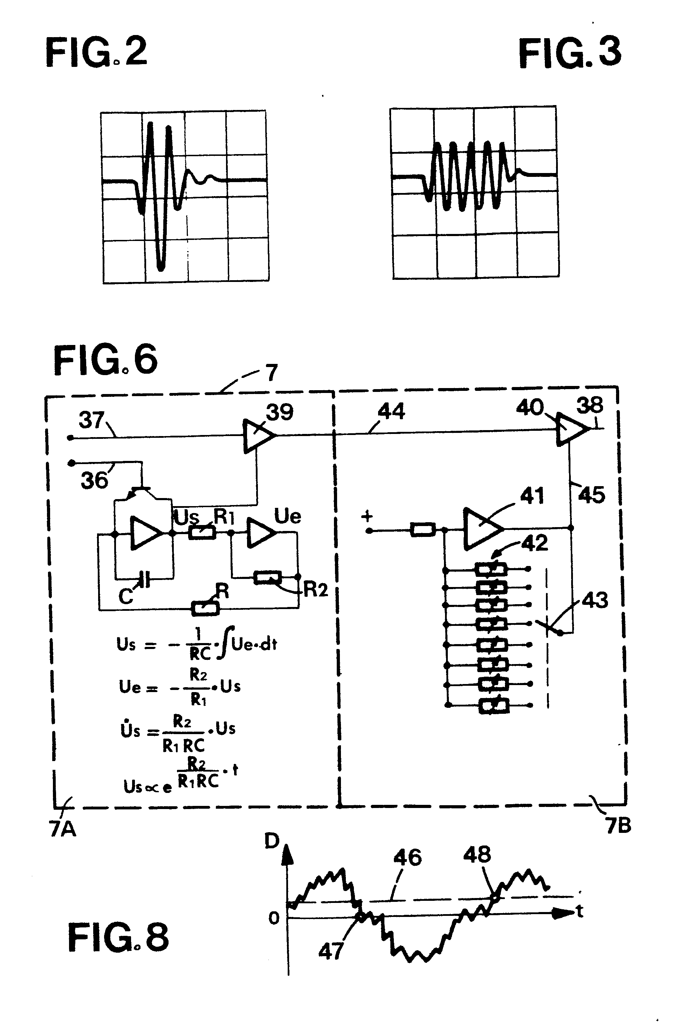

La figure 2 représente schématiquement un train d'ondes ultrasonores tel qu'il est émis par 3 sur la figure 1, tandis que la figure 3 représente un train d'ondes tel qu'il est émis par 4.

On remarquera que le train selon fig. 3 est de plus longue durée et d'amplitude moindre que celui selon fig. 2. Comme on vient de le voir, le dispositif 3 servant à la détermination du diamètre, et par conséquent de la section du vaisseau, il y a intérêt à utiliser des trains d'ondes ultrasonores brefs mais d'amplitude relativement grande. Par contre, l'émetteur-récepteur 4 servant à déterminer la vitesse d'écoulement du sang à l'intérieur du vaisseau, il est indiqué d'avoir des trains d'ondes ultrasonores suffisamment longs pour déceler de façon précise des variations de fréquence dues à l'effet Doppler par suite de la réflexion des ondes émises sur les globules sanguins.

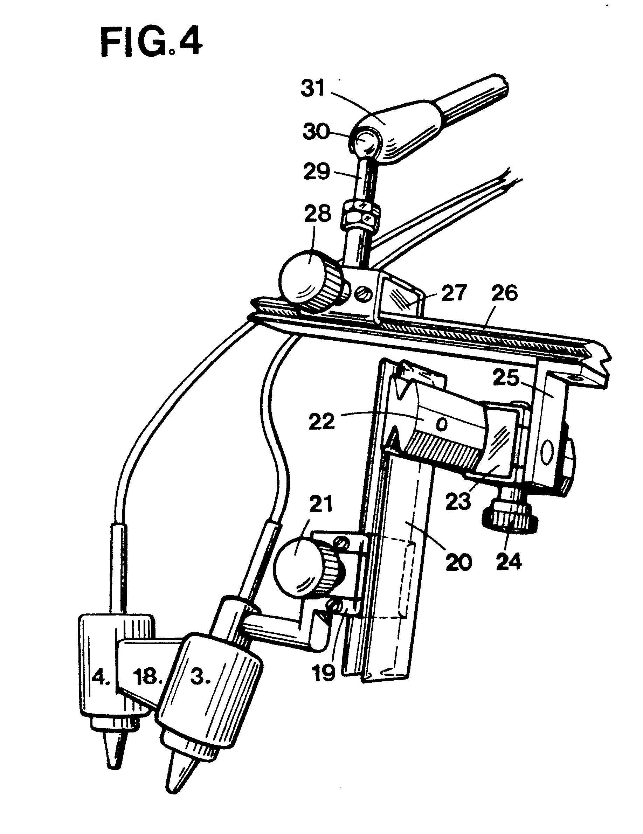

La fig. 4 montre en perspective et schématiquement la disposition des émetteurs-récepteurs 3 et 4 qui permet de les orienter de la façon qui convient aux mesures que l'on veut faire.

Les émetteurs-récepteurs 3 et 4 sont reliés rigidement entre eux par une partie 18 de façon à ce que leurs axes forment un angle constant bien déterminé. L'ensemble de 3 , 4 et 18 est relié rigidement à un coulisseau 19 prévu pour être déplacé le long d'une glissière 20 et immobilisé sur celle-ci au moyen d'une vis 21. La glissière 20 est solidaire d'une autre glissière 22 perpendiculaire et sur laquelle peut glisser un coulisseau 23. Une vis d'immobilisation du coulisseau 23 sur la glissière 22 est montrée en 24. Le coulisseau 23 est solidaire d'un bras 25 fixé rigidement à une troisième glissière 26 perpendiculaire aux deux précédentes. Sur cette troisième glissière peut glisser un coulisseau 27. Une vis d'immobilisation 28 est prévue sur ce coulisseau. Le coulisseau 27 est solidaire d'un bras 29 se terminant à son extrémité libre par une tête sphérique 30 coopérant avec un logement de même forme prévu dans un support 31. Ainsi 29 et 31 constituent une liaison à rotule à friction permettant d'amener l'ensemble des glissières et des coulisseaux dans n'importe quelle position angulaire choisie. Il s'agit d'amener le plan qui contient les axes des émetteurs-récepteurs 3 et 4 à coïncider avec l'axe du vaisseau 2 selon fig. 1, à l'endroit que l'on veut observer. Ayant amené l'ensemble selon fig. 4 dans une position approximative, on fait le réglage fin en agissant sur les différents coulisseaux des glissières de manière que l'extrémité des deux émetteurs-récepteurs 3, 4 soit en contact avec la peau et se trouve dans la position représentée sur la fig. 1. Ceci étant fait, on procède au réglage suivant qui est d'amener l'émetteur-récepteur 3 à être exactement perpendiculaire à l'axe du vaisseau 2 à l'endroit considéré. Pour cela, on fait varier l'inclinaison de l'axe de l'émetteur-récepteur 3 jusqu'à ce que la fréquence Doppler observée soit minimum et aussi voisine que possible de zéro. Cela pourra se constater au moyen d'un haut-parleur 32 branché à la sortie de 9 (fig. 1). On pourra l'observer également en constatant une vitesse minimum, voisine de zéro à la sortie de 11. Cette perpendicularité étant réalisée, le dispositif selon fig. 4 est en position de travail et on mesure le temps de transit des ultrasons émis par 3 au moyen du dispositif 14, ce qui permet de déterminer le diamètre du vaisseau 2, comme on l'a dit plus haut, en calculant la distance jusqu'à 16, puis la distance jusqu'à 17, la différence de ces deux distances donnant le diamètre. Pour cette opération l'interrupteur 15 est fermé. Bien entendu, pendant cette opération de mesure du diamètre le commutateur 6 est en position de travail de l'émetteur-récepteur 3. Le diamètre et la section du vaisseau étant déterminés, on ouvre l'interrupteur 15 et on amène le commutateur 6 en position de travail de l'émetteur-récepteur 4. Les trains d'ondes émis par 4 sont réfléchis par les globules sanguins et on peut ainsi déterminer, par le calcul, la vitesse de ces globules. En pratique, on déterminera la courbe des vitesses selon un diamètre ; les différentes vitesses individuelles ainsi trouvées, correspondant donc à des rayons différents du vaisseau, seront utilisées dans le calculateur 13 pour calculer le débit maintenant qu'on connaît la section transversale. Comme déjà dit, ce calcul est connu par des publications antérieures et il n'y a pas lieu de le développer ici.

On remarquera que l'appareil décrit jusqu'ici permet une détermination exacte du débit par le fait que l'on détermine la section transversale du conduit grâce au fait que l'émetteur-récepteur 3 est amené en position perpendiculaire à l'axe du vaisseau. En outre, l'angle que fait l'axe de l'émetteur-récepteur 4 par rapport à l'axe du conduit est parfaitement connu de par la construction, si bien que l'on peut calculer exactement les vitesses $v_1$, $v_2$, $v_3$,... correspondant aux différentes distances par rapport à l'axe.L'inconvénient des appareils connus qui a été relevé au début, à savoir l'incertitude quant à la position angulaire exacte de l'émetteur-récepteur d'ondes ultrasonores par rapport à l'axe du vaisseau est ainsi éliminé.

Dans l'exemple selon fig. 4 il y a deux émetteurs-récepteurs formant entre eux un angle constant bien déterminé qui intervient dans le calcul des vitesses.

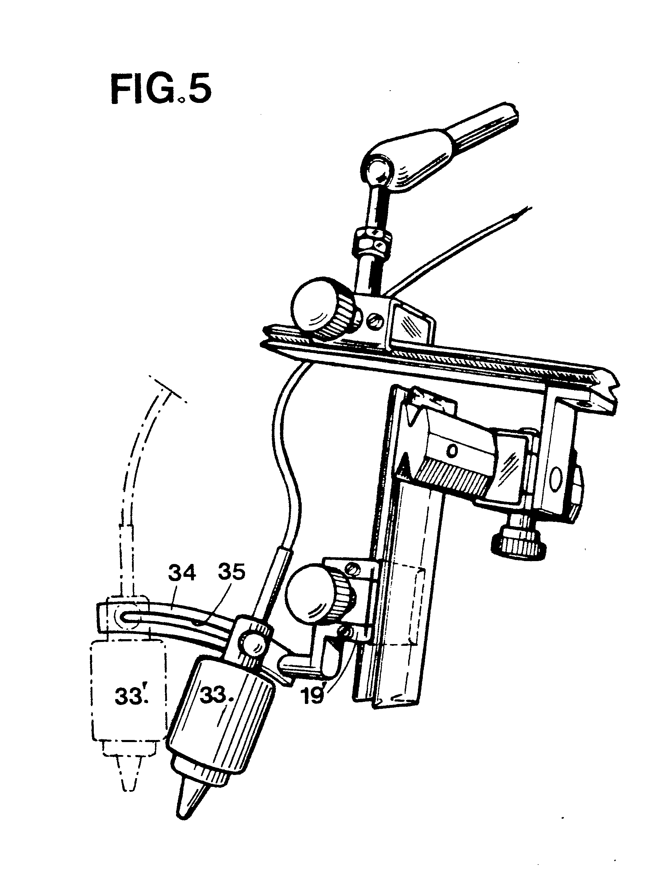

Dans la variante selon fig. 5, on a un seul émetteur-récepteur 33 monté pour glisser dans une glissière arquée 34 présentant un guide en arc de cercle 35 le long duquel on peut déplacer 33. On a représenté en trait plein 33 à l'une des extrémités de 35 et en traits mixtes en 33' ce même émetteur-récepteur à l'autre extrémité de 35. L'angle compris entre l'axe de l'émetteur-récepteur en position 33 et en position 33' correspond à l'angle compris entre les axes de 3 et 4 sur les fig. 1 et 4. Cette disposition permet de travailler avec le même émetteur-récepteur pour faire la détermination du diamètre du vaisseau (position représentée en trait plein) et pour déterminer la vitesse d'écoulement (position représentée en traits mixtes). Pour le reste, la disposition est semblable en ce sens que la glissière 34 est solidaire du coulisseau 19' qui est l'homologue de 19 sur la fig. 4. Il est inutile de décrire en détail les glissières et les autres coulisseaux car leur disposition est identique à celle de la fig. 4.

Dans les appareils connus utilisés en échographie Doppler acoustique, on est limité en ce qui concerne la profondeur des vaisseaux à l'intérieur des tissus. Les appareils connus sont utilisables, comme on l'a dit au début, pour autant que la profondeur du vaisseau soumis à examen n'excède pas 2 cm environ. Autrement l'amortissement des ultrasons du milieu dispersif que constituent les tissus du corps devient si important qu'on se heurte à des difficultés. La disposition qu'on va décrire maintenant et qui est incluse dans l'amplificateur 7 permet d'éviter cet inconvénient des appareils connus.

A l'intérieur de l'émetteur 5 il y a un générateur d'impulsions non représenté, qui fournit des impulsions périodiques déclenchant chacune un train d'ondes ultrasonores selon fig. 3. Ces impulsions de commande agissent aussi par la ligne 36 sur l'amplificateur 7, comme on va le voir.

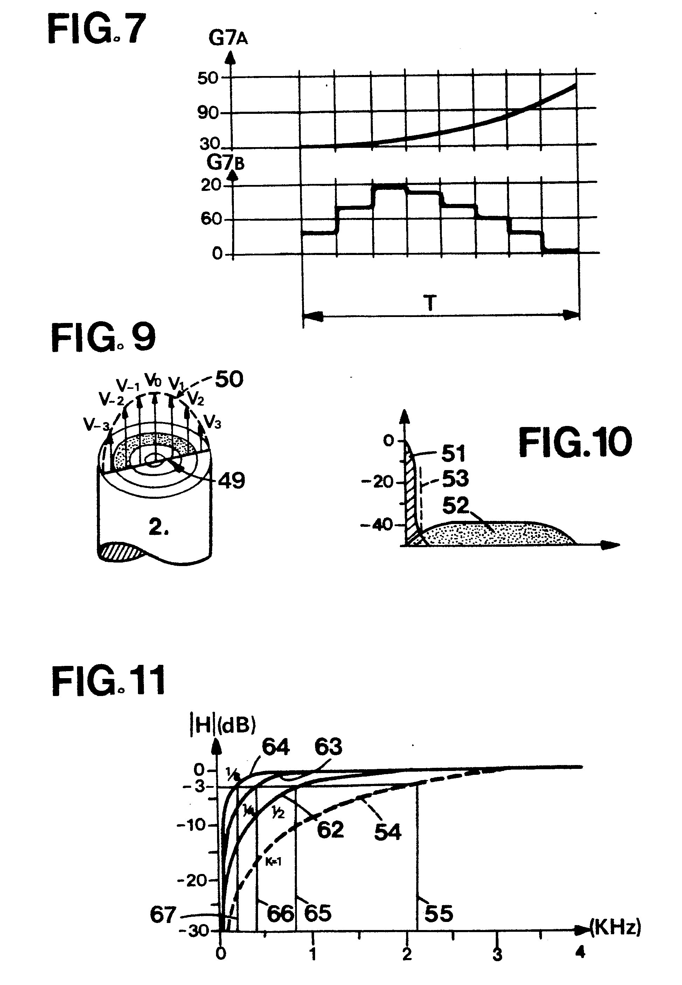

La fig. 6 montre schématiquement comment est constitué l'amplificateur 7. On voit en 36 la ligne d'amenée du signal de commande dont il vient d'être question et en 37 la ligne d'amenée de l'écho provenant de l'un ou l'autre des émetteurs-récepteurs 3, 4. La faible amplitude de l'écho provenant des globules du sang nécessite une amplification du signal revenant au transducteur 3, 4. Cette amplification a également pour but de compenser l'atténuation exponentielle subie par les ultrasons dans le milieu dispersif des tissus 1 et doit donc être dépendante de la distance parcourue par les ultrasons dans les tissus. En tenant compte de la forme exponentielle de l'atténuation, l'amplification A(t) de l'écho reçu entre deux émissions est donnée par :

$$A(t) = ht\ e^{gt} \qquad\qquad (3.3)$$

où g et h sont des constantes qui dépendent de la structure des tissus traversés par les ultrasons et peuvent donc varier d'un individu à l'autre.

Afin de conserver une certaine souplesse d'adaptation, l'amplification ht peut être modifiée par l'utilisateur. La valeur de g est déterminée à partir des coefficients d'atténuation des tissus biologiques.

L'amplification A(t), donnée par l'équation 3.3, est réalisée en deux étapes :

- amplification de la forme $e^{gt}$ : (partie 7A de 7)
- amplification de la forme ht : (partie 7B de 7).

La partie 7A de 7 représente schématiquement un dispositif électronique fournissant une amplification de la forme $e^{gt}$, c'est-à-dire une compensation exponentielle du signal arrivant en 36. 39 est un amplificateur à gain variable,
si bien qu'à la sortie de 39, on a un signal compensé comme
on vient de le dire. Ce signal arrive dans un autre amplificateur à gain variable 40, dans la partie 7B. La partie 7B
de 7 (fig. 6) comprend un amplificateur 41 et on a en 42
une série de potentiomètres qui correspondent chacun à une
certaine profondeur de tissu, jusqu'à la profondeur maximum
admise qui sera, par exemple, de 10 cm. Chacun des potentiomètres correspondant à une certaine profondeur est réglé
pour donner une résistance qui dépend de la qualité du
tissu à cette profondeur, du point de vue de sa dispersion.
Un commutateur automatique 43 balaie périodiquement les
contacts de sortie des différents potentiomètres 42 pour
envoyer à l'amplificateur 40 une succession de commandes
qui sont autant de corrections ponctuelles de la courbe
d'amplification exponentielle, si bien qu'en 38, à la sortie
de l'amplificateur 40, on obtient un signal corrigé exponentiellement et de plus optimalisé en tenant compte de l'épais
seur du tissu traversé par les ondes ultrasonores et de sa
nature.

On a indiqué au bas de la partie 7A les équations
qui permettent de comprendre comment on réalise la correctio
exponentielle au moyen du circuit indiqué en 7A.

La figure 7 représente deux diagrammes de ce qui se
passe simultanément dans les parties 7A et 7B (fig. 6)
durant une période T s'écoulant entre deux impulsions de
commande successives en 36, c'est-à-dire aussi entre deux
trains successifs selon fig. 3.

Dans la partie supérieure on a représenté en ordonnée
en G7A le gain exponentiel en 39 et dans la partie inférieur
en ordonnées également le gain G7B variable de l'amplificateur 40 correspondant à un certain réglage des potentiomètre
42. Le signal qui arrive à 40 par 44 est l'écho arrivé en
37 amplifié exponentiellement selon la courbe exponentielle

0150672

représentée dans la partie supérieure de la fig. 7.

Lorsque le commutateur 43 met en fonction successivement les différents potentiomètres 42, la tension appliquée à l'amplificateur 40 par la ligne 45 varie à chaque période selon une succession de 8 valeurs différentes dont l'allure est, par exemple, celle représentée sur la partie inférieure de la fig. 7. L'action simultanée par 44 et 45 sur l'amplificateur 40 donne en 38 un écho amplifié et qui correspond au produit des valeurs instantanées des deux courbes selon fig. 7. La courbe inférieure de la fig. 7 représente en fait le gain variable de l'amplificateur 40 sous l'effet de la commande du dispositif selon 7B. Ainsi, par l'action simultanée des deux dispositifs selon 7A et 7B, on obtient en 38 une amplification optimalisée de l'écho en complétant la correction exponentielle de l'amortissement des trains d'ondes dans le milieu dispersif par une amplification supplémentaire du signal Doppler que l'on règle au moyen des potentiomètres 42 pour diverses distances parcourues par ces ondes et en faisant varier l'amplification de ce signal à chaque période d'approximativement zéro jusqu'à une valeur pour laquelle les passages par zéro de la courbe représentant le signal soient nets et permettent le comptage de ces passages. Ainsi, on peut déterminer exactement la fréquence Doppler et la vitesse d'écoulement pour chacune de ces distances ou profondeurs dans le tissu.

L'écho provenant de cibles en mouvement (globules sanguins par exemple) possède une fréquence différente de celle de l'onde incidente (effet Doppler). La variation de fréquence est fonction de la vitesse des cibles. Il s'agit donc dans l'échographie Doppler de déterminer la fréquence Doppler pour en déduire la vitesse des cibles. Si on représente graphiquement la fréquence Doppler relevée par échographie, on obtient du fait du "bruit" inévitable une courbe périodique quelque peu irrégulière dont les passages par zéro ne sont pas nets et peuvent même être multiples dans une même zone. Il en résulte une inexactitude que l'appareil selon l'invention vise à éliminer. Pour cela, on cherche à réaliser des passages par zéro qui soient nets de façon à pouvoir les compter avec exactitude et en déduire la valeur réelle de la fréquence Doppler et donc la vitesse du sang.

Le signal Doppler amplifié arrivant en 38 doit avant de faire l'objet d'un comptage de passages à zéro, être démodulé, ce qui est fait par le démodulateur 8 qui est de type connu. Le signal Doppler démodulé arrive en 9 où il est d'une part digitalisé par un circuit électronique connu et où il donne lieu à une séparation des échos dont il sera question plus loin. La sortie de 9 agit sur le détecteur de zéro 10.

La fig. 8 représente schématiquement comment travaille le détecteur de zéro 10. Sur cette figure, on a représenté en abscisse le temps t et en ordonnée le signal Doppler D sortant de 9. Ce signal varie périodiquement comme illustré sur la fig. 8, les dents de scie illustant l'effet du "bruit On voit que les passages à zéro ne sont pas univoques et pour permettre un comptage sans équivoque le dispositif 10 introduit un seuil de sensibilité représenté par la droite horizontale 46 distante de l'axe des abscisses d'une quantit supérieure à l'amplitude du "bruit". Ainsi, tout ce qui est au-dessus de la ligne 46 sera considéré comme positif par le dispositif 10, tout ce qui est au-dessous de l'axe des abscisses sera considéré comme négatif et ce qui est compris entre cet axe et 46 ne sera pas pris en considération. De cette façon, les passages par zéro pourront être comptés de façon précise. Ainsi, sur la fig. 8 on aura au point 47 un passage par zéro du positif au négatif, au point 48 un passage par zéro du négatif au positif.

En pratique, le seuil de la ligne 46 est fixé à l'aide d'un comparateur que l'on règle en fonction du
niveau du "bruit".

La fig. 9 illustre le profil des vitesses du sang
dans une section transversale du vaisseau 2 selon fig. 1.
Le cylindre représenté correspond au diamètre intérieur du
vaisseau. La section transversale a été divisée en un
certain nombre d'anneaux plus une partie centrale circulaire. Le long d'un diamètre quelconque tel que 49, à chaque
instant, les vitesses ont des valeurs qui sont fonction du
rayon. Ainsi, par exemple, au centre la vitesse sera maximum et égale à $v_0$. Pour le premier anneau à partir du centre,
la vitesse sera $v_1$ d'un côté du centre et $v_{-1}$ de l'autre
côté. Pour l'anneau suivant $v_2$ et $v_{-2}$ et pour l'anneau
suivant $v_3$ et $v_{-3}$. La courbe pointillée 50 représente le
profil de la vitesse selon un diamètre du vaisseau 2. Le
calculateur 11 calcule à chaque instant les vitesses instantanées représentées sur la fig.9 et ce sont ces vitesses qui
sont fournies au calculateur de débit 13 qui, par ailleurs,
reçoit de 14 la valeur du diamètre. Le calculateur fait
aussi le calcul des sections des différents demi-anneaux
représentés sur la fig. 9. On considère, en effet, des demi-
anneaux car les vitesses $v_1$, $v_2$, $v_3$ ne sont pas forcément
égales aux vitesses $v_{-1}$, $v_{-2}$, $v_{-3}$, si la surface intérieure
du vaisseau n'a pas les mêmes qualités (colmatage par exemple) en des endroits symétriques.

Lors de l'échographie Doppler acoustique pulsée à
canaux multiples, on se trouve en présence d'un phénomène
qui complique les choses. Ce phénomène provient du fait que
les parois d'un conduit élastique tel qu'un vaisseau sanguin
changent périodiquement de diamètre sous l'effet des variations de pression du liquide à son intérieur, donc lors des
pulsations sanguines. Ces variations de diamètre produisent
un effet Doppler différent de celui qu'on utilise pour
déterminer la vitesse du sang et le débit sanguin et elles
se manifestent au voisinage des parois lors de leur vibration. Il y a intérêt à séparer l'effet Doppler découlant de la vitesse du sang de l'effet Doppler résultant du mouvement des parois. Cette séparation permet, en effet, de déterminer avec un maximum d'exactitude le profil des vitesses jusqu'au voisinage immédiat des parois, tandis que sans cette séparation, dans la région voisine des parois, on aurait une évaluation inexacte des vitesses du fait de la superposition des deux effets Doppler. De plus, cette séparation permet par l'examen de l'effet Doppler dû au mouvement des parois de déduire des informations utiles quant à l'état de ces parois. En effet, l'examen des variations de diamètre du vaisseau par l'effet Doppler permet de tirer des conclusions quant à l'élasticité et à l'état physiologique des parois (degré de calcification par exemple).

La fig. 10 représente le spectre de fréquences de l'écho démodulé. En abscisse on a représenté la fréquence Doppler et en ordonnée l'amplitude en décibels de l'écho. On constate que la partie hachurée voisine de l'axe des ordonnées et représentée en 51 correspond à l'effet Doppler de la paroi dont on vient de parler. La surface représentée en ponctué le long de l'axe des abscisses et désignée par 52 représente l'effet Doppler dû au mouvement du sang dans le vaisseau considéré.

Si l'on désire séparer les effets Doppler 51 et 52, on peut imaginer d'employer un filtre idéal dont la fréquence de coupure correspondrait à la ligne verticale 53. Si l'on veut étudier le signal Doppler relatif à la vitesse du sang, on utilisera un filtre passe-haut et si l'on veut au contraire étudier l'effet Doppler dû à la paroi élastique on utilisera un filtre passe-bas. En pratique on n'arrive pas à obtenir cette coupure idéale en 53. On pourrait améliorer les résultats obtenus avec un filtre du premier ordre en employant un filtre d'ordre supérieur, ce qui serait beaucoup plus compliqué et beaucoup plus onéreux, mais sans obtenir pour autant une coupure selon 53.

La figure 11 représente la fonction de transfert d'un filtre passe-haut visant à isoler l'effet Doppler dû à la vitesse du sang. La ligne pointillée 54 représente cette fonction de transfert dans le cas d'un filtre du premier ordre.

Si l'on fait, comme il est d'usage, la coupure à -3dB, on voit que toute la partie de la courbe pointillée 54 qui est à gauche de l'ordonnée 55 est perdue, donc que les vitesses correspondantes ne sont pas tenues en compte. Vu l'importance du déchet, cette solution est mauvaise. L'appareil selon fig. 1 vise à diminuer considérablement ce déchet en fréquence et en vitesse de la façon suivante.

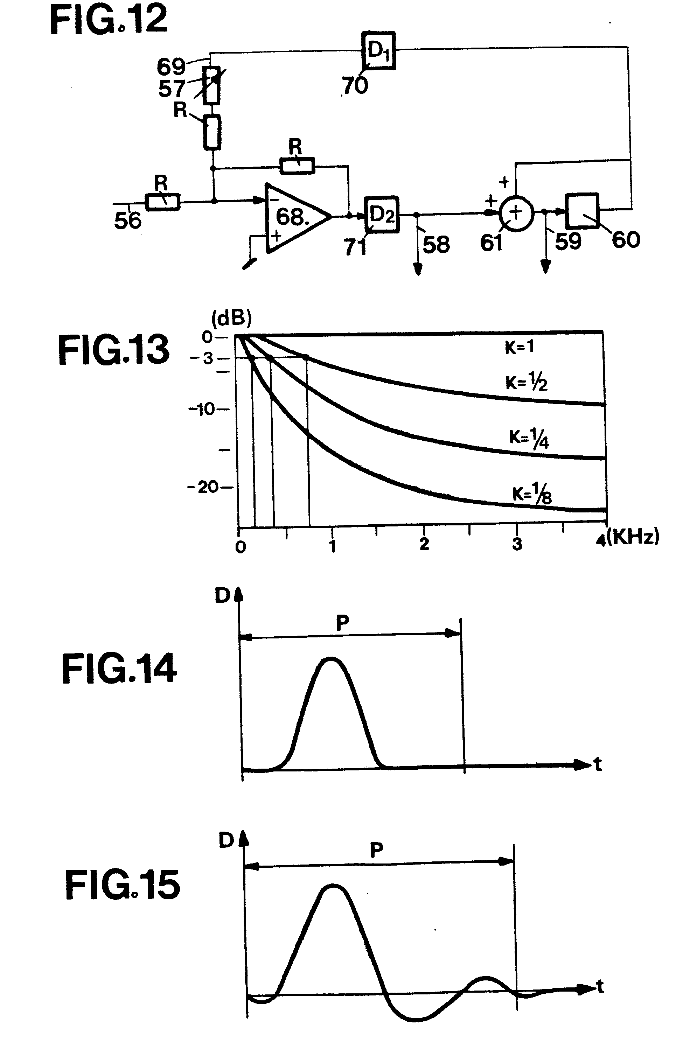

La fig. 12 représente schématiquement les éléments que contient le dispositif 9 de la fig. 1.

Sur la fig. 12, 56 représente l'arrivée du démodulateur 8. 70 est un convertisseur digital-analogique ($D_1$) et 71 est un convertisseur analogique-digital ($D_2$). L'élément 57 sera défini plus loin, ainsi que sa fonction. Tous les autres éléments visibles sur la fig. 12 constituent un filtre passe-haut et passe-bas classiques du premier ordre.

La sortie passe-haut est en 58 et la sortie passe-bas en 59. On a désigné en 60 un élément de retard d'une période égale au laps de temps séparant deux émissions successives d'ondes ultrasonores. Cet élément est destiné à conserver le signal pendant une période, en vue de l'introduire d'une part dans un additionneur 61 et de l'envoyer d'autre part à l'élément 57. Cet élément 57 a pour fonction d'introduire un facteur de filtrage inférieur à l'unité, pour changer la réponse en fréquence du filtre et amener la limite de la bande passante du filtre au voisinage immédiat de la fréquence Doppler maximum de l'écho de la paroi. En se référant à la fig. 11, on voit que pour un facteur de filtrage égal à 1, on a affaire à la courbe 54 dont on a parlé, qui correspond à l'absence d'élément 57. Pour un facteur de filtrage égal à 1/2 la courbe 54 se trouve remplacée par une courbe 62, pour un facteur de filtrage égal à 1/4 par une courbe 63 et pour un facteur de filtrage égal à 1/8 par une courbe 64. On voit que ces différentes courbes 62, 63, 64 ont pour effet que la coupure (pour -3dB) se situe considérablement plus à gauche que la ligne 55. On a indiqué cette coupure par les lignes verticales 65, 66 et 67. L'élément 57 est constitué simplement par une résistance. La valeur de cette résistance déterminera le coefficient 1/2, 1/4, 1/8 ou autre. On voit qu'ainsi il est possible, par adjonction de cet élément 57, d'introduire dans un filtre du premier ordre un facteur de filtrage K inférieur à l'unité, ce qui améliore considérablement la qualité du filtre. 68 est un amplificateur en montage inverseur utilisé pour réaliser une somme pondérée des signaux 56 et 69.

Sur le graphique fig. 11, l'échelle des abscisses correspond aux fréquences Doppler exprimées en KHz et la valeur maximum est indiquée comme étant 4. Si la fréquence avec laquelle les trains d'ondes ultrasonores sont émises est de 8KHz, ce graphique correspond dans son étendue maximum à la moitié de la fréquence. On voit donc que le filtre du premier ordre amélioré par l'élément 57 permet d'obtenir de façon simple et extrêmement économique une séparation des effets correspondants à 51 et 52 se rapprochant de ce que donnerait un filtre idéal, comme indiqué en 53.

La fig. 13 est analogue à la fig. 11 mais se rapporte au cas d'un filtre passe-bas (sortie 59). Sur ce graphique on voit qu'un filtre classique du premier ordre (K = 1) laisse tout passer. Par contre, la coupure à -3dB donne des valeurs de plus en plus favorables quand K diminue comme on le voit pour les valeurs 1/2, 1/4, 1/8.

La fig. 14 représente le débit sanguin D de l'artère fémorale pendant une pulsation de durée P, tel qu'on peut l'établir en utilisant, comme il est connu, un filtre passe-haut classique du premier ordre, tandis que la fig.

représente ce même débit tel qu'on le détermine au moyen d'un filtre du premier ordre dans lequel on a introduit, comme décrit plus haut, un facteur de filtrage K inférieur à l'unité.

On trouve la théorie des filtres et, en particulier, des filtres du premier ordre dans l'article de H. Urkowitz "Analysis and synthesis of delay line periodic filters" IRE Trans. Circuit Theory CT-4, 41, 1957.

La comparaison des fig. 14 et 15 montre l'avantage considérable qu'il y a à introduire, comme on l'a décrit, un facteur de filtrage K inférieur à l'unité. Le reflux postsystolique ainsi que le flux diastolique qui correspondent à des fréquences Doppler de faible valeur sont en effet coupés lorsque K=1, comme c'est le cas dans les filtres classiques du premier ordre.

Bien que l'exemple illustré aux dessins ait été décrit en référence à du sang circulant dans un vaisseau sanguin, il est bien évident que l'invention n'est pas limitée à un tel usage mais peut s'appliquer dans d'autres cas d'un fluide circulant dans un conduit d'une façon générale.

REVENDICATIONS

1. Procédé de détermination par échographie Doppler de la vitesse d'écoulement et du débit d'un fluide dans un conduit, caractérisé en ce qu'on détermine la position d'un plan perpendiculaire au conduit (2) en faisant tourner selon différentes orientations un dispositif comprenant un émetteur-récepteur (3) de trains d'ondes jusqu'à observation d'un effet Doppler minimum, en ce qu'on détermine la dimension du conduit (2) selon cette direction perpendiculaire, par mesure du temps de transit entre l'émission d'un train d'ondes et la réception de ce train après réflexion sur les parois (16, 17) du conduit, lorsque la direction de propagation de ces trains est perpendiculaire à la direction du conduit, en ce qu'on oriente un émetteur-récepteur (4) de trains d'ondes selon une deuxième direction faisant un angle prédéterminé avec la première et située dans un plan passant par la dite perpendiculaire et par l'axe du conduit (2) à l'endroit du dit plan perpendiculaire, et en ce qu'on détermine par mesures d'effet Doppler le profil des vitesses du fluide selon cette deuxième direction.

2. Procédé selon la revendication 1, caractérisé en ce qu'on utilise pour l'émission dans la première direction des trains d'ondes de durée inférieure et d'amplitude supérieure, par rapport à la durée et à l'amplitude des trains d'ondes selon la seconde direction.

3. Appareil pour la mise en oeuvre du procédé selon la revendication 1, caractérisé en ce qu'il comporte un dispositif comprenant au moins un émetteur-récepteur (3, 4) de trains d'ondes, orientable par rapport à un conduit (2) dans lequel s'écoule le fluide, et des moyens (14) pour mesurer le temps de transit entre l'émission d'un train d'ondes et la réception de ce train réfléchi par les parois du conduit, en vue de déterminer une orientation d'émission perpendiculaire à l'axe du conduit (2), et des moyens (11) pour déterminer par mesure d'effet Doppler, le profil des vitesses du fluide selon une direction faisant un angle prédéterminé avec la dite direction perpendiculaire.

4. Appareil selon la revendication 3, caractérisé en ce qu'il comprend deux émetteurs- récepteurs (3,4 fig. 4) solidaires, montés pour tourner ensemble selon différentes directions et dont les axes d'émission-réception font entre eux un angle constant, prédéterminé.

5. Appareil selon la revendication 4, caractérisé en ce que l'un (3) des émetteurs-récepteurs (3,4), qui sert à déterminer la dimension du conduit, émet des trains d'ondes de durée inférieure et d'amplitude supérieure, par rapport aux trains d'ondes émis par le second (4) émetteur-récepteur, qui sert à déterminer le profil des vitesses.

6. Appareil selon la revendication 3, caractérisé en ce qu'il comporte un seul émetteur-récepteur (33) monté sur un support orientable (fig. 5), cet émetteur-récepteur étant mobile sur ce support entre deux positions d'émission-réception faisant entre elles un angle prédéterminé.

7. Appareil selon la revendication 6, caractérisé en ce qu'il comporte des moyens (3,4) pour faire émettre des trains d'ondes d'une certaine amplitude et d'une certaine durée pour la détermination de la dimension du conduit, et d'une amplitude moindre mais d'une durée supérieure lors de la détermination du profil des vitesses.

# FIG. 1

Emetteur 5.

6

Emetteurs -récepteurs

4 3

37 36

1.

16

2.

$V_1$
$V_2$
$V_3$

17

| Mesure du temps de transit 14. |

Amplification optimalisée 7.

15

38

diamètre

Démodulation 8.

Calculateur de débit 13.

56

32

Digitalisation

Séparation des échos 9.

vitesse

Calculateur de vitesse 11.

Détecteur des zéros 10.

Calcul amplitude mouvement paroi 12.

# FIG.2

# FIG.3

# FIG.6

7

37

36

39

44

40 38

45

$U_s$ $R_1$ $U_e$

41

42

$C$

$R$

$R_2$

43

$$U_s = -\frac{1}{RC} \cdot \int U_e \cdot dt$$

$$U_e = -\frac{R_2}{R_1} \cdot U_s$$

$$\mathring{U}_s = \frac{R_2}{R_1 RC} \cdot U_s$$

$$U_s \propto e^{\frac{R_2}{R_1 RC} \cdot t}$$

7A

7B

+

D

46

48

0

t

47

# FIG.8

# FIG.4

FIG.5

**FIG. 7**

**FIG. 9**

**FIG. 10**

**FIG. 11**

6/6

0150672

**FIG.12**

**FIG.13**

**FIG.14**

**FIG.15**

0150672

Office européen
des brevets

**RAPPORT DE RECHERCHE EUROPEENNE**

Numéro de la demande

EP 84 81 0623

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.⁴) |
|---|---|---|---|
| A | FR-A-2 334 957 (SIEMENS)<br>* Page 2, lignes 13-20 *<br><br>--- | 1,3 | G 01 F 1/66<br>G 01 P 5/00<br>A 61 B 8/06 |
| A | US-A-4 370 985 (H. TAKEICHI et al.)<br>* Figure 1; abrégé *<br><br>--- | 1,3 | |
| A | US-A-4 336 808 (M. OHNO et al.)<br>* Figure 2; abrégé *<br><br>----- | 1,3 | |

**DOMAINES TECHNIQUES RECHERCHES (Int. Cl.⁴)**

A 61 B
G 01 F
G 01 P

Le présent rapport de recherche a été établi pour toutes les revendications

| Lieu de la recherche<br>LA HAYE | Date d'achèvement de la recherche<br>13-03-1985 | Examinateur<br>MIELKE W |
|---|---|---|